(19) Europäisches Patentamt
European Patent Office
Office européen des brevets

(11) **EP 3 494 431 B1**

(12) **EUROPÄISCHE PATENTSCHRIFT**

(45) Veröffentlichungstag und Bekanntmachung des Hinweises auf die Patenterteilung:
**25.06.2025 Patentblatt 2025/26**

(21) Anmeldenummer: **17751667.1**

(22) Anmeldetag: **25.07.2017**

(51) Internationale Patentklassifikation (IPC):
**G02B 27/00** (2006.01)    **G02B 23/24** (2006.01)
**A61B 1/00** (2006.01)

(52) Gemeinsame Patentklassifikation (CPC):
**A61B 1/00193; A61B 1/00096; A61B 1/0011; G02B 23/2415; G02B 23/2423; G02B 23/2484; G02B 27/0018**

(86) Internationale Anmeldenummer:
**PCT/EP2017/068779**

(87) Internationale Veröffentlichungsnummer:
**WO 2018/024548 (08.02.2018 Gazette 2018/06)**

(54) **OPTISCHES SYSTEM EINES STEREO-VIDEOENDOSKOPS MIT SEITLICHER BLICKRICHTUNG SOWIE VERFAHREN ZUM HERSTELLEN DESSELBEN**

OPTICAL SYSTEM OF A STEREO-VIDEO ENDOSCOPE HAVING A LATERAL VIEWING DIRECTION, AND METHOD FOR PRODUCING SAME

SYSTÈME OPTIQUE D'UN STÉRÉO-VIDÉO-ENDOSCOPE POURVU D'UN CHAMP DE VISION LATÉRAL ET PROCÉDÉ DE FABRICATION DE CE DERNIER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.08.2016 DE 102016214272**

(43) Veröffentlichungstag der Anmeldung:
**12.06.2019 Patentblatt 2019/24**

(73) Patentinhaber: **Olympus Winter & Ibe GmbH 22045 Hamburg (DE)**

(72) Erfinder:
• **SCHOUWINK, Peter 22926 Ahrensburg (DE)**
• **ZHAO, Jianxin 22399 Hamburg (DE)**

(74) Vertreter: **Seemann & Partner Patentanwälte mbB Raboisen 6 20095 Hamburg (DE)**

(56) Entgegenhaltungen:
EP-A- 2 730 210          DE-A- 102013 215 422
DE-A- 102014 206 513     JP-A- H09 288 240
US-A- 5 861 987

EP 3 494 431 B1

**Beschreibung**

Die Erfindung betrifft ein optisches System eines Stereo-Videoendoskops mit seitlicher Blickrichtung, umfassend eine seitwärts blickende distale optische Baugruppe und eine proximale optische Baugruppe, wobei die proximale optische Baugruppe einen linken Linsensystemkanal und einen rechten Linsensystemkanal umfasst, die gleichartig aufgebaut sind, und wobei die distale optische Baugruppe dazu eingerichtet ist, aus einem Objektraum einfallendes Licht in den linken Linsensystemkanal und in den rechten Linsensystemkanal der proximalen optischen Baugruppe einzukoppeln, und wobei die distale optische Baugruppe in einer Lichteinfallsrichtung aufeinanderfolgend eine Eintrittslinse, eine Umlenkprismengruppe und eine Austrittslinse umfasst, wobei die Umlenkprismengruppe in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst.

Ferner betrifft die Erfindung ein Stereo-Videoendoskop mit seitlicher Blickrichtung sowie ein Verfahren zum Herstellen eines optischen Systems eines Stereo-Videoendoskops mit seitlicher Blickrichtung, wobei das Stereo-Videoendoskop eine seitwärts blickende distale optische Baugruppe und eine proximale optische Baugruppe umfasst, und wobei die proximale optische Baugruppe einen linken Linsensystemkanal und einen rechten Linsensystemkanal umfasst, die gleichartig aufgebaut sind, und wobei die distale optische Baugruppe dazu eingerichtet ist, aus einem Objektraum einfallendes Licht in den linken Linsensystemkanal und in den rechten Linsensystemkanal der proximalen optischen Baugruppe einzukoppeln, und wobei die distale optische Baugruppe in einer Lichteinfallsrichtung aufeinanderfolgend eine Eintrittslinse, eine Umlenkprismengruppe und eine Austrittslinse umfasst, wobei die Umlenkprismengruppe in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst.

Videoendoskope, bei denen das an einer distalen Spitze eines Endoskopschafts eintretende Licht durch ein optisches System auf einen oder mehrere Bildsensoren gelenkt wird, sind in verschiedenen Ausführungen bekannt. Es gibt Endoskope mit Geradeausblick, einer sog. 0°-Blickrichtung, Endoskope mit (fester) seitlicher Blickrichtung sowie Endoskope mit verstellbarer Blickrichtung (auch als V-DOV-Endoskope bezeichnet).

Außerdem sind Stereo-Videoendoskope bekannt, die dazu eingerichtet sind, ein stereoskopisches Bildpaar und/oder zwei stereoskopische Videokanäle aufzunehmen. Mit solchen Instrumenten ist es möglich, ein 3D-Abbild eines distal vor dem Ende des Endoskopschafts liegenden Objekts in einem Untersuchungs- oder Operationsraum zu erzeugen.

Stereo-Videoendoskope mit seitlicher Blickrichtung sind seitwärts blickende Endoskope mit einem festen vom Geradeausblick abweichenden Blickwinkel. Solche Endoskope umfassen vielfach eine Prismenanordnung aus mehreren Prismen, die die aus dem Objektraum unter einem Winkel zur Längsachse des Endoskopschafts in das optische System eintretenden Lichtstrahlen zweimal reflektieren und seitenrichtig in Richtung des Endoskopschafts umlenken. Ein solches Endoskop ist beispielsweise aus der DE 10 2014 206 513 A1 des Anmelders Olympus Winter & Ibe, Hamburg, bekannt.

Eine Umlenkprismenanordnung eines solchen Stereo-Videoendoskops umfasst typischerweise zwei oder drei Prismen. Die Prismen sind an ihren gemeinsamen Grenzflächen vielfach miteinander verkittet. Bei einer solchen Umlenkprismenanordnung findet die Reflektion der einfallenden Lichtbündel an zwei sowohl zur optischen Achse der Eintrittslinse als auch zur Längsachse des Endoskopschafts schrägstehenden reflektierenden Grenzflächen eines zweiten Prismas statt. Das zweite Prisma der Umlenkprismenanordnung befindet sich in Lichteinfallsrichtung hinter einem ersten Prisma, welches unmittelbar hinter der Eintrittslinse angeordnet ist. Die schrägstehende reflektierende Grenzfläche des zweiten Prismas, an der die zweite Reflexion stattfindet, bildet teilweise eine gemeinsame Grenzfläche mit dem ersten Prisma, welches die einfallenden Lichtstrahlen zuerst durchlaufen.

Die Eintrittslinse des optischen Systems eines solchen Stereo-Videoendoskops definiert die optische Achse des optischen Systems. Das optische System umfasst Blenden oder Menisken, die ein Blickfeld bzw. die Öffnungswinkel der Optik festlegen. Innerhalb des Blickfeldes in das optische System einfallende Lichtbündel werden von dem optischen System auf einen oder mehrere Bildsensoren abgebildet. Lichtbündel, die von außerhalb des Blickfeldes in das optische System einfallen, führen vielfach zu Reflexionen innerhalb des optischen Systems und erzeugen so sog. "Geisterbilder" oder "Flares".

Eine bekannte Umlenkprismengruppe, in der solche Geisterbilder entstehen können, umfasst ein erstes Prisma und ein zweites Prisma, die miteinander verkittet sind. Das erste Prisma weist eine Eintrittsseite und eine Austrittsseite auf, wobei die Eintrittsseite gegenüber der Austrittsseite geneigt ist. Die Austrittsseite des ersten Prismas grenzt unmittelbar an eine zweite Eintrittsseite des zweiten Prismas an. Beispielsweise sind das erste und das zweite Prisma an diesen beiden Seiten miteinander verkittet. Das zweite Prisma umfasst ferner eine Reflexionsseite und eine zweite Austrittsseite. Licht, welches aus dem Bildfeld in die Umlenkprismengruppe einfällt, durchquert die Eintrittsseite des ersten Prismas und tritt an dessen Austrittsseite wieder aus. Das Licht gelangt anschließend unmittelbar durch die zweite Eintrittsseite in das zweite Prisma, wird an der Reflexionsseite innerhalb des zweiten Prismas reflektiert und verlässt dieses an der Austrittsseite.

Ein unter einem großen Winkel zur optischen Achse der Eintrittslinse in die Optik einfallender peripherer Lichtstrahl gelangt durch die Eintrittslinse in das erste Prisma und durchquert dessen Eintrittsseite und Austrittsseite. Gleichzeitig mit der ersten Austrittsseite durchquert der Lichtstrahl auch die zweite Eintrittsseite des zweiten Prismas. Wie bereits erwähnt, können diese beiden Prismenflächen miteinander verkittet sein. Der Lichtstrahl wird nun an der Reflexionsseite des zweiten Prismas reflektiert und trifft im spitzen Winkel auf die gemeinsame Grenzfläche zwischen dem ersten und zweiten Prisma, also von der Rückseite her auf die zweite Eintrittsseite des zweiten Prismas. Dort erfährt der Lichtstrahl Fresnelreflexion oder Totalreflexion und wird zurück zur Reflexionsseite des zweiten Prismas reflektiert. Von dort aus gelangt er erneut zu der zweiten Eintrittsseite des zweiten Prismas und wird erneut beispielsweise unter Totalreflexion von der Innenseite her an dieser Grenzfläche reflektiert. Anschließend gelangt der Lichtstrahl in einen linken oder einen rechten Linsensystemkanal und erzeugt dort ein Geisterbild. Diese an sich bekannte Vierfachreflexion in der Umlenkprismengruppe ist unerwünscht.

Aus DE 10 2013 215 422 A1 ist ein optisches System eines Stereo-Videoendoskops mit seitlicher Blickrichtung bekannt, welches als Eintrittslinse einen erhabenen negativen Meniskus aufweist. Das optische System umfasst eine Umlenkprismenanordnung mit Grenzflächen, an denen eine Reflexion der im Strahlengang geführten Lichtbündel erfolgt. Hierzu sind die Grenzflächen verspiegelt.

In EP 2 730 210 A1 ist ein Endoskop mit einem hochauflösenden Bildaufnehmer gezeigt. Dieses Endoskop umfasst eine Umlenkprismeneinheit, in der einfallendes Licht zunächst an einer ersten Reflexionsfläche und anschließend an einer zweiten Reflexionsfläche reflektiert wird. Eine Totalreflexion ist ausdrücklich nur im Zusammenhang mit der Reflexion an der zweiten Grenzfläche erwähnt.

US 5 861 987 A zeigt ein Stereo-Endoskop mit einem röhrenförmigen länglichen Einschubteil, in dem ein optisches Objektsystem angeordnet ist. Im distalen Abschnitt ist ein schrägblickendes Prisma angeordnet, das aus zwei Teilprismen aufgebaut ist, die einfallendes Licht in Richtung der optischen Achse umlenken.

JP H09288240 A zeigt ein optisches System eines Endoskops, dass zur Änderung der Blickrichtung eingerichtet ist. Das System umfasst ein Prisma und ein optisches Element. Durch Reflektion an den Seiten und des Prismas wird einfallendes Licht in Richtung des optischen Elements reflektiert. Eine der Seitenflächen ist teilweise mit einer Metallbeschichtung versehen.

Aufgabe der Erfindung ist es, ein optisches System eines Stereo-Videoendoskops mit fester seitlicher Blickrichtung, ein Stereo-Videoendoskop mit fester seitlicher Blickrichtung sowie ein Verfahren zum Herstellen und zum Reparieren eines optischen Systems eines Stereo-Videoendoskops mit fester seitlicher Blickrichtung anzugeben, welches unempfindlicher gegenüber von außerhalb des Blickfelds einfallenden Lichtbündeln ist, insbesondere im Hinblick auf Geisterbilder.

Die Aufgabe wird gelöst durch ein optisches System eines Stereo-Videoendoskops mit fester seitlicher Blickrichtung, umfassend eine seitwärts blickende distale optische Baugruppe und eine proximale optische Baugruppe, wobei die proximale optische Baugruppe einen linken Linsensystemkanal und einen rechten Linsensystemkanal umfasst, die gleichartig aufgebaut sind, und wobei die distale optische Baugruppe dazu eingerichtet ist, aus einem Objektraum einfallendes Licht in den linken Linsensystemkanal und in den rechten Linsensystemkanal der proximalen optischen Baugruppe einzukoppeln, und wobei die distale optische Baugruppe in einer Lichteinfallsrichtung aufeinanderfolgend eine Eintrittslinse, eine Umlenkprismengruppe und eine Austrittslinse umfasst, wobei die Umlenkprismengruppe in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst, wobei die erste Eintrittsseite und die Reflexionsseite einen Winkel einschließen, der größer als ein Totalreflexionswinkel des zweiten Prismas ist, wobei eine erste Teilfläche der Reflexionsseite des zweiten Prismas mit einer reflektierenden Schicht versehen ist und eine zweite Teilfläche der Reflexionsseite wahlweise unbeschichtet ist oder mit einer Antireflexbeschichtung versehen ist, wobei sich die erste und die zweite Teilfläche zur Gesamtfläche der Reflexionsseite ergänzen, wobei das optische System dadurch fortgebildet ist, dass ein Abstand zwischen der ersten Eintrittsseite und der Reflexionsseite in der ersten Teilfläche stets größer ist als ein Abstand zwischen der ersten Eintrittsseite und der Reflexionsseite in der zweiten Teilfläche.

In das optische System einfallende Lichtbündel, welche aus einem Bildfeld im Objektraum in das optische System einfallen, durchqueren die erste Eintrittsseite des ersten Prismas, werden an der Grenzfläche des Prismenkörpers abgelenkt und gelangen zur ersten Austrittsseite des ersten Prismas. Die erste Austrittsseite des ersten Prismas ist mit der zweiten Eintrittsseite des zweiten Prismas beispielsweise verkittet. Die Lichtbündel gelangen über diese Grenzfläche zur zweiten Eintrittsseite des zweiten Prismas. Sie werden an der Grenzfläche des Prismenkörpers abgelenkt und gelangen zur rückwärtigen Reflexionsseite des zweiten Prismas. Von dort aus werden die Lichtbündel zur zweiten Eintrittsseite des zweiten Prismas zurückreflektiert und erfahren innerhalb des Prismenkörpers des zweiten Prismas an dieser Grenzfläche Totalreflexion im Prismenkörper. Die Lichtbündel werden also an einer Innenseite der zweiten Eintrittsseite reflektiert. Von dort aus verlassen die Lichtbündel die Umlenkprismengruppe durch die Austrittsseite des zweiten Prismas.

Unter einem "Totalreflexionswinkel" wird im Kontext der vorliegenden Beschreibung der Grenzwinkel der Totalreflexion verstanden. Dieser Winkel ist durch das Material des ersten und zweiten Prismas bestimmt, genauer durch den Brechungsindex der verwendeten Materialien. Das erste Prisma und das zweite Prisma sind insbesondere aus identischem Material hergestellt, beispielsweise aus der gleichen Glassorte.

Die Berechnung des Totalreflexionswinkels $\Theta c$ erfolgt beispielsweise anhand der Formel

$$\Theta_c = \arcsin(n1/n2),$$

wobei n1 = 1 für Luft gilt und n2>1 der Brechungsindex des Materials der Prismen ist.

Es handelt sich um den Brechungsindex, wie er im Snellius'schem Brechungsgesetz verwendet wird. Es wird also nicht der komplexe Brechungsindex verwendet, der zur Berücksichtigung der Absorption der Welle im Medium als komplexe Zahl angegeben wird.

Es ist ferner insbesondere vorgesehen, dass der linke Linsensystemkanal eine linke optische Achse und der rechte Linsensystemkanal eine rechte optische Achse aufweist. Die linke optische Achse und die rechte optische Achse sind insbesondere parallel zueinander ausgerichtet.

Unter einer "seitlichen Blickrichtung" oder dem Begriff "seitwärts blickend" wird im Kontext der vorliegenden Beschreibung das Folgende verstanden. Das Stereo-Videoendoskop weist einen Schaft auf.

Dieser Schaft ist starr oder flexibel. Bei starrem Schaft weist dieser eine Längserstreckungsrichtung auf. Bei flexiblem Schaft erstreckt sich der Schaft in einem distalen Endbereich in einer Längserstreckungsrichtung. Die Blickrichtung des Endoskops schließt mit dieser Längserstreckungsrichtung einen Winkel ein, der von Null verschieden ist. Dieser Winkel ist konstant. Beispielsweise beträgt ein solcher Winkel 30°.

In dem optischen System gemäß Aspekten der Erfindung ist eine Vierfachreflexion an den Grenzflächen der Umlenkprismengruppe, wie sie aus dem Stand der Technik an sich bekannt ist, vorteilhaft ausgeschlossen. Bei Stereo-Videoendoskopen muss die Reflexionsfläche des zweiten Prismas größer als bei Prismen von Endoskopen sein, welche keine stereoskopische Abbildung liefern. Dies ist erforderlich, da für den rechten und linken Stereokanal eine möglichst große Stereobasis realisiert werden soll. Eine große Stereo-Basis erlaubt es, einen großen 3D-Effekt zu erzeugen. Diese konstruktive Anforderung führt jedoch zu der erwähnten Gefahr von mehrfachen Reflexionen, insbesondere der beschriebenen Vierfachreflexion. Diese Reflexionen erzeugen unerwünschte Geisterbilder. Durch die Anordnung der ersten Eintrittsseite und der Reflexionsseite im beschriebenen Winkel werden solche (Mehrfach-)Reflexionen wirksam unterdrückt.

Gemäß einer vorteilhaften Ausführungsform ist das optische System dadurch fortgebildet, dass das optische System derart eingerichtet ist, dass aus einem Blickfeld des optischen Systems einfallende Lichtstrahlen unter einem Winkel auf die Reflexionsseite des zweiten Prismas treffen, der größer als der Totalreflexionswinkel ist.

In diesem Zusammenhang ist insbesondere vorgesehen, dass das optische System zumindest eine Blende umfasst, die das Blickfeld des optischen Systems begrenzt, wobei der Winkel zwischen der ersten Eintrittsseite und der Reflexionsseite unter Berücksichtigung eines Materials des zweiten Prismas, welches einen Brechungsindex und somit einen Totalreflexionswinkel festlegt, und unter Berücksichtigung eines maximalen Blickwinkels, der durch das Blickfeld festgelegt ist, derart eingestellt ist, dass für alle aus dem Blickfeld einfallenden Lichtstrahlen an der Reflexionsseite des zweiten Prismas Totalreflexion stattfindet.

Durch diese Optimierung der Verhältnisse zwischen dem durch Blenden oder Menisken gesteuerten Einfallswinkel auf die erste Eintrittsseite des ersten Prismas werden alle aus dem Innenraum des Bildfeldes in das optische System einfallenden Lichtbündel an der Reflexionsseite des zweiten Prismas totalreflektiert.

Das optische System ist dadurch fortgebildet, dass eine erste Teilfläche der Reflexionsseite des zweiten Prismas mit einer reflektierenden Schicht versehen ist und eine zweite Teilfläche der Reflexionsseite wahlweise unbeschichtet ist oder mit einer Antireflexbeschichtung versehen ist, wobei sich die erste und die zweite Teilfläche zur Gesamtfläche der Reflexionsseite ergänzen.

Als Antireflexbeschichtung ist beispielsweise eine Beschichtung vorgesehen, wie sie bei der Vergütung von fotografischen Optiken zum Einsatz kommt. Dort werden Antireflexbeschichtungen eingesetzt, um den Reflexionsgrad der optischen Oberflächen von Linsen, Objektiven, Prismen oder Platten zu verringern und die Transmission zu erhöhen. Bei Objektiven und Okularen mit einer solchen Beschichtung spricht man vielfach von einer Vergütung, bei Brillen oder Sichtfenstern vielfach von einer Entspiegelung.

Die reflektierende Schicht ist insbesondere auf einer äußeren Oberfläche der Reflexionsseite des zweiten Prismas aufgebracht. Es handelt sich um eine reflektierende Beschichtung, beispielsweise eine aufgedampfte Schicht aus Silber (Ag) oder Aluminium (Al). Die reflektierende Beschichtung sorgt dafür, dass Lichtstrahlen an einer inneren Oberfläche, also im Prismenkörper, reflektiert werden. Durch den nicht reflektierend beschichteten Teil der Reflexionsseite treten Lichtstrahlen aus der Reflexionsseite des zweiten Prismas aus. Es handelt sich um diejenigen Lichtbündel, die unter großen Winkeln von außerhalb des Bildfeldes in das optische System eintreten und bei herkömmlichen optischen Systemen zu den typischen Vierfachreflexionen führen. Bei dem optischen System gemäß Aspekten der Erfindung wird die Entstehung von Geisterbildern vorteilhaft verhindert oder unterdrückt.

Es ist ferner vorgesehen, dass ein Abstand zwischen der ersten Eintrittsseite und der Reflexionsseite in der ersten Teilfläche stets größer ist als ein Abstand zwischen der ersten Eintrittsseite und der Reflexionsseite in der zweiten Teilfläche. Die nicht reflektierend beschichtete zweite Teilfläche der Reflexionsseite liegt also in einem unteren engen bzw. schmalen Bereich des zweiten Prismas.

Ferner ist das optische System vorteilhaft dadurch fortgebildet, dass aus einem Objektraum einfallende erste Lichtstrahlen in der ersten Teilfläche der Reflexionsfläche reflektiert und in den linken Linsensystemkanal eingekoppelt werden und aus dem Objektraum einfallende zweite Lichtstrahlen in der zweiten Teilfläche der Reflexionsfläche reflektiert und in den rechten Linsensystemkanal eingekoppelt werden.

Beispielsweise definiert die optische Achse der Eintrittslinse eine Lage einer Trennlinie zwischen den Teilflächen. Diese Trennlinie kann außerdem beispielsweise 50%, 40%, 30% oder 20% vom unteren Rand entfernt liegen, jeweils Bezug nehmend auf die gesamte Höhe der Fläche in dieser Richtung.

Die Aufgabe wird ferner gelöst durch ein Stereo-Videoendoskop mit fester seitlicher Blickrichtung, welches dadurch fortgebildet ist, dass es ein optisches System nach einem oder mehreren der zuvor genannten Ausführungsformen umfasst.

Auf das Stereo-Videoendoskop treffen gleiche oder ähnliche Vorteile zu, wie sie bereits im Hinblick auf das optische System selbst erwähnt wurden. Vorteilhaft treten nämlich bei einem solchen Stereo-Videoendoskop auch bei Lichtbündeln, die unter großen Winkeln in dessen optisches System einfallen, keine Geisterbilder auf.

Die Aufgabe wird ferner gelöst durch ein Verfahren zum Herstellen eines optischen Systems eines Stereo-Videoendoskops mit fester seitlicher Blickrichtung, wobei das Stereo-Videoendoskop eine seitwärts blickende distale optische Baugruppe und eine proximale optische Baugruppe umfasst, und wobei die proximale optische Baugruppe einen linken Linsensystemkanal und einen rechten Linsensystemkanal umfasst, die gleichartig aufgebaut sind, und wobei die distale optische Baugruppe dazu eingerichtet ist, aus einem Objektraum einfallendes Licht in den linken Linsensystemkanal und in den rechten Linsensystemkanal der proximalen optischen Baugruppe einzukoppeln, und wobei die distale optische Baugruppe in einer Lichteinfallsrichtung aufeinanderfolgend eine Eintrittslinse, eine Umlenkprismengruppe und eine Austrittslinse umfasst, wobei die Umlenkprismengruppe in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst, wobei das erste und das zweite Prisma so ausgewählt oder angeordnet werden, dass die erste Eintrittsseite und die Reflexionsseite einen Winkel einschließen, der größer als ein Totalreflexionswinkel des zweiten Prismas ist, wobei eine erste Teilfläche der Reflexionsseite des zweiten Prismas mit einer reflektierenden Schicht versehen wird und eine zweite Teilfläche der Reflexionsseite wahlweise unbeschichtet belassen wird oder mit einer Antireflexbeschichtung versehen wird, wobei sich die erste und die zweite Teilfläche zur Gesamtfläche der Reflexionsseite ergänzen, wobei dieses Verfahren dadurch fortgebildet ist, dass ein Abstand zwischen der ersten Eintrittsseite und der Reflexionsseite in der ersten Teilfläche stets größer ist als ein Abstand zwischen der ersten Eintrittsseite und der Reflexionsseite in der zweiten Teilfläche.

Die Auswahl erfolgt derart, dass das optische System so eingerichtet ist, dass aus einem Bildfeld des optischen Systems einfallende Lichtstrahlen unter einem Winkel auf die Reflexionsseite des zweiten Prismas treffen, der größer als der Totalreflexionswinkel ist. Im Übrigen treffen auch für das Verfahren gleiche oder ähnliche Vorteile zu, wie sie bereits im Hinblick auf das optische System selbst erwähnt wurden, so dass Wiederholungen vermieden werden sollen.

Das Verfahren ist insbesondere dadurch fortgebildet, dass das optische System mit zumindest einer Blende versehen wird, die das

Blickfeld des optischen Systems begrenzt, wobei der Winkel zwischen der ersten Eintrittsseite und der Reflexionsseite unter Berücksichtigung eines Materials des zweiten Prismas, welches einen Brechungsindex und somit einen Totalreflexionswinkel festlegt, und unter Berücksichtigung eines maximalen Blickwinkels, der durch das Blickfeld festgelegt ist, derart eingestellt wird, dass für alle aus dem Blickfeld einfallenden Lichtstrahlen an der Reflexionsseite des zweiten Prismas Totalreflexion stattfindet.

Schließlich wird die Aufgabe gelöst durch ein Verfahren zum Reparieren eines Stereo-Videoendoskops mit seitlicher Blickrichtung, wobei ein optisches System des Stereo-Videoendoskops eine seitwärts blickende distale optische Baugruppe und eine proximale optische Baugruppe umfasst, und wobei die proximale optische Baugruppe einen linken Linsensystemkanal und einen rechten Linsensystemkanal umfasst, die gleichartig aufgebaut sind, und wobei die distale optische Baugruppe dazu eingerichtet ist, aus einem Objektraum einfallendes Licht in den linken Linsensystemkanal und in den rechten Linsensystemkanal der proximalen optischen Baugruppe einzukoppeln, und wobei die distale optische Baugruppe in einer Lichteinfallsrichtung aufeinanderfolgend eine Eintrittslinse, eine Umlenkprismengruppe und eine Austrittslinse umfasst, wobei die Umlenkprismengruppe in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst, wobei die Umlenkprismengruppe ausgetauscht und durch eine neue Umlenkprismengruppe ersetzt wird, bei der das erste und das zweite Prisma so ausgewählt oder angeordnet werden, dass die erste Eintrittsseite und die Reflexionsseite einen Winkel einschließen, der größer als ein Totalreflexionswinkel des zweiten Prismas ist, wobei eine erste Teilfläche der Reflexionsseite des zweiten Prismas mit einer reflektierenden Schicht versehen wird und eine zweite Teilfläche der Reflexionsseite wahlweise unbeschichtet belassen wird oder mit einer Antireflexbeschichtung versehen wird, wobei sich die erste und die zweite Teilfläche zur Gesamtfläche der Reflexionsseite ergänzen, wobei dieses Verfahren dadurch fortgebildet ist, dass ein Abstand zwischen der ersten Eintrittsseite und der Reflexionsseite in der ersten Teilfläche stets größer ist als ein Abstand zwischen der ersten Eintrittsseite und der Reflexionsseite in der zweiten Teilfläche.

Durch das Verfahren zum Reparieren des Stereo-Videoendoskops wird vorteilhaft die Möglichkeit geschaffen, vorhandene Endoskope im Hinblick auf die Anfälligkeit für Geisterbilder zu verbessern.

Im Kontext der vorliegenden Beschreibung wird unter einer Beschichtung der Reflexionsseite stets eine reflektierende Beschichtung, beispielsweise mit Silber (Ag) oder Aluminium (Al), verstanden. Ist also die Reflexionsseite unbeschichtet oder teilweise unbeschichtet, so weist die Reflexionsseite keine reflektierende Beschichtung oder teilweise keine reflektierende Beschichtung auf.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1 ein Stereo-Videoendoskop in schematisch vereinfachter Darstellung,

Fig. 2 eine schematisch vereinfachte Darstellung eines optischen Systems eines Stereo-Videoendoskops gemäß dem Stand der Technik in einer Schnittansicht,

Fig. 3 eine schematisch vereinfachte Darstellung des optischen Systems eines Stereo-Videoendoskops, wobei der Strahlengang des linken Linsensystemkanals dargestellt ist, und

Fig. 4 eine schematisch vereinfachte Darstellung des optischen Systems eines Stereo-Videoendoskops, wobei der Strahlengang des rechten Linsensystemkanals dargestellt ist.

In den Zeichnungen sind jeweils gleiche oder gleichartige Elemente und/oder Teile mit denselben Bezugsziffern versehen, so dass von einer erneuten Vorstellung jeweils abgesehen wird.

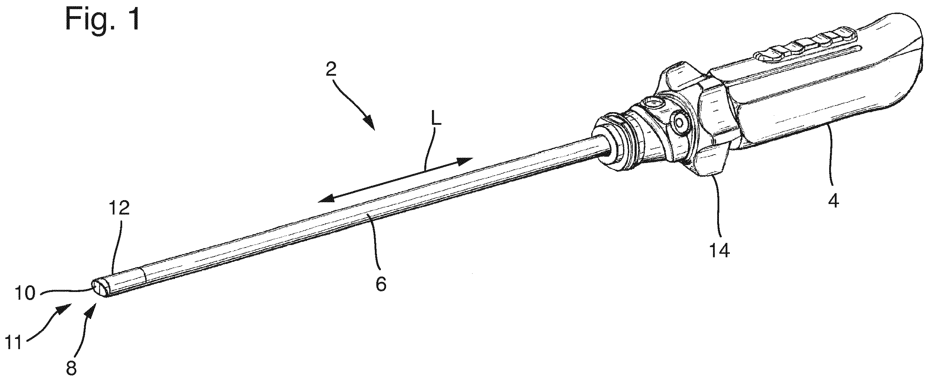

Fig. 1 zeigt in schematisch vereinfachter perspektivischer Darstellung ein Stereo-Videoendoskop 2, umfassend einen proximalen Handgriff 4, an den sich ein beispielsweise starrer Endoskopschaft 6 anschließt. Der Endoskopschaft 6 kann ebenso flexibel oder halbflexibel sein. An einer distalen Spitze 8 des Endoskopschafts 6 befindet sich ein Eintrittsfenster 10, durch welches Licht aus einem Objektraum 11, beispielsweise aus einem Operations- und/oder Beobachtungsfeld, in ein in Fig. 1 nicht sichtbares optisches System des Stereo-Videoendoskops 2 eintritt. Das optische System des Stereo-Videoendoskops 2 ist beispielsweise in einem distalen Abschnitt 12 des Endoskopschafts 6 angeordnet. Das optische System bildet Objekte, die sich im Objektraum 11 befinden, auf Bildsensoren ab. Diese Bildsensoren sind beispielsweise solche mit hoher Auflösung, z.B. HD, 4K oder folgende Technologien.

Das gezeigte Stereo-Videoendoskop 2 ist ein chirurgisches Instrument. Außerdem handelt es sich um ein Endoskop mit fester seitlicher Blickrichtung. Das Eintrittsfenster 10 ist geneigt im Endoskopschaft 6 montiert, so dass eine optische Achse der Eintrittslinse des nicht dargestellten optischen Systems mit einer Längserstreckungsrichtung L des Endoskopschafts 6 des Stereo-Videoendoskops 2 einen festen Winkel einschließt. Dieser Winkel beträgt beispielsweise zwischen 10° und 30°.

Eine Änderung der Blickrichtung um eine Längsachse des Endoskopschafts 6 wird durch eine Drehung des Handgriffs 4 bewirkt. Das im distalen Abschnitt 12 vorhandene optische System rotiert bei dieser Drehung des Handgriffs 4 mit. Zur Beibehaltung der Horizontallage des angezeigten Bildes wird bei einer Drehung des Handgriffs 4 ein Drehrad 14 festgehalten. Dies bewirkt, dass die Bildsensoren im Inneren des Endoskopschafts 6 die Drehbewegung nicht mitvollziehen.

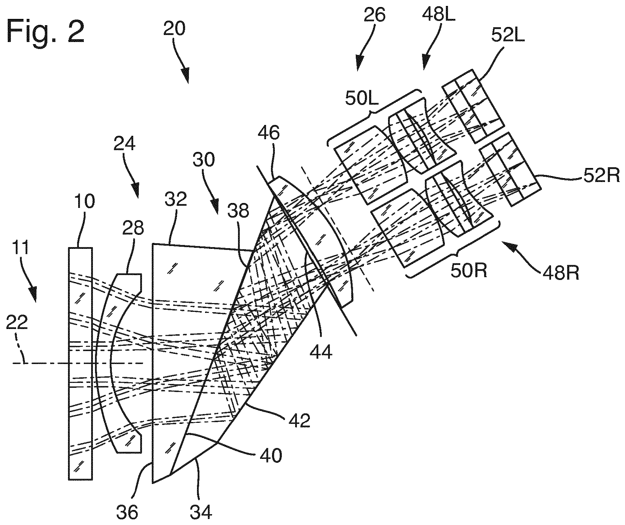

Fig. 2 zeigt ein optisches System 20, wie es in Stereo-Videoendoskopen 2 gemäß dem Stand der Technik zum Einsatz kommt.

Das optische System 20 definiert die feste seitliche Blickrichtung des Stereo-Videoendoskops 2. Die optische Achse 22 schließt mit der Längserstreckungsrichtung L des Endoskopschafts 6 einen festen Winkel von beispielsweise 30° ein. Das optische System 20 umfasst eine seitwärts blickende distale optische Baugruppe 24 und eine proximale optische Baugruppe 26. Aus dem Objektraum 11 durch das Eintrittsfenster 10 einfallendes Licht trifft zunächst auf die Eintrittslinse 28 und gelangt anschließend in eine Umlenkprismengruppe 30 der distalen optischen Baugruppe 24. Die Umlenkprismengruppe 30 umfasst in Lichteinfallsrichtung aufeinanderfolgend ein erstes Prisma 32 und ein zweites Prisma 34.

In Lichteinfallsrichtung durchqueren die Lichtbündel, welche die Eintrittslinse 28 verlassen, zunächst eine erste Eintrittsseite 36 des ersten Prismas 32. Die Lichtbündel durchqueren den Körper des ersten Prismas 32 und gelangen zu dessen erster Austrittsseite 38. Die erste Austrittsseite 38 ist gegenüber der ersten Eintrittsseite 36 geneigt. Das erste Prisma 32 und das zweite Prisma 34 sind beispielsweise miteinander verkittet. Das zweite Prisma 34 umfasst eine zweite Eintrittsseite 40, durch welche das aus dem ersten Prisma 32 durch dessen erste Austrittsseite 38 austretende Licht in das zweite Prisma 34 eintritt. Die erste Austrittsseite 38 des ersten Prismas 32 und die zweite Eintrittsseite 40 des zweiten Prismas 34 sind im dargestellten Beispiel miteinander verkittet. Das zweite Prisma 34 umfasst ferner eine Reflexionsseite 42, welche gegenüber der zweiten Eintrittsseite 40 geneigt ist. Die über die zweite Eintrittsseite 40 in das zweite Prisma 34 eintretenden Lichtbündel werden an der Reflexionsseite 42 des zweiten Prismas 34 reflektiert. Von dort aus treffen sie von der Rückseite her auf die zweite Eintrittsseite 40 des zweiten Prismas 34. Die Eintrittsseite 40 ist beispielsweise in einem oberen Bereich, in welchem sie nicht an die Austrittsseite 38 des ersten Prismas 32 grenzt, mit einer reflektierenden Beschichtung beschichtet. Ferner ist alternativ zum Verkitten des ersten und zweiten Prismas 32, 34 vorgesehen, dass zwischen der Austrittsseite 38 des ersten Prismas 32 und der Eintrittsseite 40 des zweiten Prismas 34 eine Maske angeordnet ist. Diese Maske bewirkt einen Luftspalt zwischen der Austrittsseite 38 und der Eintrittsseite 40, so dass die von innen an der Eintrittsseite 40 reflektierten Lichtstrahlen an der Grenzfläche Glas-Luft totalreflektiert werden. Die Lichtbündel werden an der Rückseite der Eintrittsseite 40 unter einem solchen Winkel reflektiert, dass sie anschließend das zweite Prisma 34 an seiner zweiten Austrittsseite 44 verlassen. Von dort aus gelangen die Lichtbündel weiter in Lichteinfallsrichtung zu einer Austrittslinse 46 der distalen optischen Baugruppe 24.

Die proximale optische Baugruppe 26 umfasst einen linken Linsensystemkanal 48L und einen rechten Linsensystemkanal 48R. Der linke und der rechte Linsensystemkanal 48L, 48R sind gleichartig oder gar identisch aufgebaut. Sie sind ferner so angeordnet, dass eine in Fig. 2 nicht dargestellte linke optische Achse und eine ebenfalls nicht dargestellte rechte optische Achse des linken bzw. rechten Linsensystemkanals 48L, 48R parallel zueinander ausgerichtet sind. Der linke Linsensystemkanal 48L umfasst eine abbildende linke Linsengruppe 50L, die das einfallende Licht auf einen linken Bildsensor 52L abbildet. Entsprechend umfasst der rechte Linsensystemkanal 48R eine abbildende rechte Linsengruppe 50R, die das einfallende Licht auf einen rechten Bildsensor 52R abbildet.

Die distale optische Baugruppe 24 ist dazu eingerichtet, die aus dem Objektraum 11 einfallenden Lichtbündel sowohl in den linken Linsensystemkanal 48L als auch in den rechten Linsensystemkanal 48R einzukoppeln.

Bei Endoskopen, wie sie aus dem Stand der Technik bekannt sind, ist die Reflexionsseite 42 des zweiten Prismas 34 vollflächig mit einer reflektierenden Beschichtung versehen. Beispielsweise ist die Außenseite des zweiten Prismas 34 an der Reflexionsseite 42 mit Aluminium (Al) oder Silber (Ag) bedampft.

Die Fläche der Reflexionsseite 42 des zweiten Prismas 34 ist bei Stereo-Videoendoskopen wesentlich größer als bei Endoskopen, welche keine stereoskopischen Abbildungen liefern. Dies ist erforderlich, um einen möglichst großen Abstand des linken und rechten Stereokanals zu ermöglichen. Eine solche große Stereobasis ermöglicht einen starken 3D-Effekt.

Eine solche Prismenkonstruktion geht jedoch mit dem technischen Nachteil einher, dass es schnell zu Mehrfachreflexionen kommt, die ein sog. Geisterbild erzeugen. Solche Geisterbilder werden von peripheren Lichtstrahlen erzeugt, die unter großem Winkel zur optischen Achse 22 aus dem Objektraum 11 in das optische System 20 einfallen.

Ein solcher peripherer Lichtstrahl gelangt durch die Eintrittslinse 28 in das erste Prisma 32 und von dort aus in das zweite Prisma 34. Er trifft auf die Reflexionsseite 42 des zweiten Prismas 34, wird dort reflektiert und trifft im spitzen Winkel auf die Grenzfläche zwischen dem ersten und dem zweiten Prisma 32, 34. Von dort aus wird er zurück zur Reflexionsseite 42 des zweiten Prismas 34 reflektiert und gelangt erneut zur zweiten Eintrittsseite 38 des zweiten Prismas 34. An dieser Grenzfläche erfolgt erneut Totalreflexion, so dass der Lichtstrahl anschließend über die Austrittsseite 44 die Umlenkprismengruppe 30 verlässt und in dem linken oder rechten Linsensystemkanal 48L, 48R ein Geisterbild erzeugt.

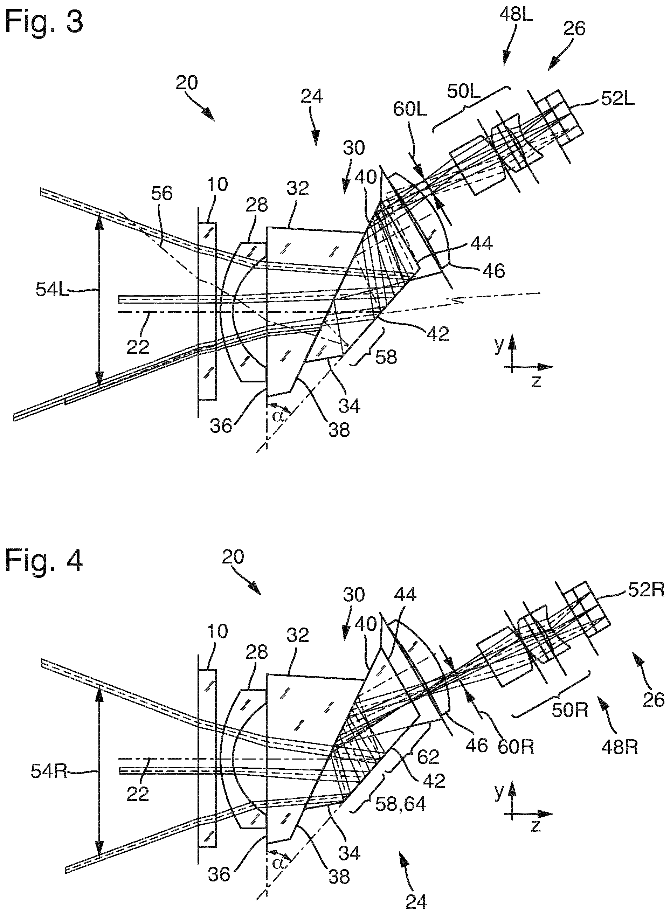

Fig. 3 zeigt ein optisches System 20 in einer schematisch vereinfachten Darstellung gemäß einem Ausführungsbeispiel. In dem optischen System ist lediglich aus Gründen der Vereinfachung der Darstellung nur der linke Linsensystemkanal 48L dargestellt.

Fig. 4 zeigt eine entsprechende Darstellung eines optischen Systems 20 gemäß einem Ausführungsbeispiel, wobei in dieser Darstellung der rechte Linsensystemkanal 48R dargestellt ist.

Fig. 3 zeigt den Strahlengang von Lichtbündeln, welche in den linken Linsensystemkanal fallen, entsprechend zeigt Fig. 4 den Strahlengang von Lichtbündeln, welche in den rechten Linsensystemkanal 48R fallen. Die Lichtstrahlen, welche in den linken Linsensystemkanal abgebildet werden, stammen aus einem Sichtfeld des linken Kanals 54L, die Lichtstrahlen, die in den rechten Linsensystemkanal 48R abgebildet werden, stammen aus einem Sichtfeld des rechten Kanals 54R.

In Fig. 3 ist beispielhaft ein Lichtstrahl 56 gezeigt, der unter einem großen Winkel zur optischen Achse 22 in das optische System 20 einfällt. Er gelangt in einen Bereich des zweiten Prismas 34, der auch als "B-Down-Bereich" bezeichnet werden soll. Dieser B-Down-Bereich 58 wird von den Lichtbündeln, die in den linken Linsensystemkanal 48L abgebildet werden, typischerweise nicht genutzt. Die Lichtstrahlen, welche in den rechten Linsensystemkanal fallen (vgl. Fig. 4) werden hingegen im B-Down-Bereich 58 der Reflexionsseite 42 des zweiten Prismas 34 reflektiert. So kann es vorkommen, dass der Lichtstrahl 56, welcher im B-Down-Bereich 58 des zweiten Prismas 34 reflektiert wird, in den rechten Linsensystemkanal 48R gelangt und dort ein Geisterbild erzeug.

Um unter anderem dieses Phänomen zu unterdrücken oder vollständig zu beseitigen, ist das optische System 20 derart eingerichtet, dass die erste Eintrittsseite 36 des ersten Prismas 32 und die Reflexionsseite 42 des zweiten Prismas 34 einen Winkel α einschließen, der größer als ein Totalreflexionswinkel $\theta_C$ des zweiten Prismas 34 ist.

Der Totalreflexionswinkel $\theta_C$ wird anhand der bekannten Formel

$$\Theta_C = arcsin\ (n1/n2)$$

berechnet, wobei n1 = 1 für Luft gilt und n2>1 der Brechungsindex des Materials des zweiten Prismas 34 ist.

Das erste und das zweite Prisma 32, 34 sind insbesondere aus dem gleichen oder identischen Material hergestellt. Zumindest werden für die beiden Prismen 32, 34 Materialien mit zumindest näherungsweise gleichem Brechungsindex verwendet. Beispielsweise werden die beiden Prismen 32, 34 aus dem gleichen Glas hergestellt.

Der Brechungsindex des Materials ist der Brechungsindex, wie er im Snellius'schen Brechungsgesetz verwendet wird. Es handelt sich also nicht um den komplexen Brechungsindex.

Das optische System 20 ist derart eingerichtet, dass die aus einem Blickfeld 54L, 54R in das optische System 20 einfallenden Lichtstrahlen unter einem solchen Winkel auf die Reflexionsseite 42 des zweiten Prismas 34 treffen, der größer als der Totalreflexionswinkel ist. So ist es möglich, dass die zweite Teilfläche 64 der Reflexionsseite 42 des zweiten

Prismas 34 unbeschichtet verbleiben kann, also nicht mit einer reflektierenden Schicht wie beispielsweise Al oder Ag bedampft ist. Es ist ebenso vorgesehen, dass die zweite Teilfläche 64 der Reflexionsseite 42 des zweiten

Prismas 34 mit einer Antireflexbeschichtung versehen ist, wie sie beispielsweise von fotografischen Optiken her bekannt ist.

Es ist ferner insbesondere vorgesehen, dass das optische System 20 zumindest eine Blende 60R, 60L umfasst, die das Blickfeld 54L, 54R des optischen Systems 20 begrenzt. Im dargestellten Ausführungsbeispiel sind separate Blenden 60L, 60R für den linken und den rechten Linsensystemkanal 48L, 48R vorgesehen, nämlich die linke Blende 60L und die rechte Blende 60R.

Der Winkel α zwischen der ersten Eintrittsseite 36 des ersten Prismas 32 und der Reflexionsseite 42 des zweiten Prismas 34 wird unter Berücksichtigung eines Materials des zweiten Prismas 34, welches einen Brechungsindex n2 und somit einen Totalreflexionswinkel $\theta_C$ festlegt, und unter Berücksichtigung eines maximalen Blickwinkels, der durch das Blickfeld 54L, 54R festgelegt ist, derart eingestellt, dass für alle aus dem Blickfeld 54L, 54R einfallenden Lichtstrahlen an der Reflexionsseite 42 des zweiten Prismas 34 Totalreflexion stattfindet.

Im Umkehrschluss bedeutet dies, dass Lichtstrahlen, die von außerhalb des Blickfeldes 54L, 54R in das optische System 20 einfallen, wie beispielsweise der Lichtstrahl 56, der in herkömmlichen Systemen ein Geisterbild verursacht, an der zweiten Teilfläche 64 der Reflexionsseite 42 des zweiten Prismas 34 nicht total reflektiert werden. Solche Lichtstrahlen verlassen das optische System 20, genauer die zweite Teilfläche 64 der Reflexionsseite 42 des zweiten Prismas 34, und werden beispielsweise auf einer geschwärzten Innenseite eines das optische System 20 aufnehmenden Tubus absorbiert.

Durch Optimierung des Verhältnisses zwischen den durch die Menisken und Blenden 60R, 60L gesteuerten Einfallswinkel auf die Reflexionsseite 42 des zweiten Prismas 34 für alle Strahlen, welche von innerhalb der Sichtfelder 54L, 54R einfallen, kann Totalreflexion an der Reflexionsseite 42 erzeugt werden. Beispielsweise muss ein Einfallswinkel auf die erste Eintrittsseite 36 des ersten Prismas 32 -7,6° betragen (das verwendete Minuszeichen bedeutet dabei in der Darstellung der Figuren eine Drehung im Uhrzeigersinn), wobei der Winkel α=36° beträgt und als Material für die Prismen S-LAH 58 verwendet wird. In einem solchen Fall werden alle auf die zweite Teilfläche 64 der Reflexionsseite 42 auftreffenden Lichtstrahlen totalreflektiert. Auf eine Beschichtung dieser Fläche kann komplett verzichtet werden oder die Fläche wird mit einer Antireflexionsbeschichtung versehen.

Gemäß der beanspruchten Erfindung ist vorgesehen, dass eine erste Teilfläche 62 der Reflexionsseite 42 des zweiten Prismas 34 mit einer reflektierenden Schicht versehen ist und eine zweite Teilfläche 64 der Reflexionsseite 42 unbeschichtet ist oder mit einer Antireflexbeschichtung versehen ist. Die erste und die zweite Teilfläche 62, 64 sind beispielhaft in Fig. 4 dargestellt. Ferner entspricht beispielhaft die zweite Teilfläche 64 dem B-Down-Bereich 58. Die erste und die zweite Teilfläche 62, 64 ergänzen sich erfindungsgemäß zur Gesamtfläche der Reflexionsseite 42. So wird vorteilhaft die Möglichkeit geschaffen, dass Lichtstrahlen 56, welche in herkömmlichen Systemen Geisterbilder verursachen, im B-Down-Bereich 58 die Reflexionsseite 42 des zweiten Prismas 34 verlassen.

Die erste und die zweite Teilfläche 62, 64 sind erfindungsgemäß ferner so auf der Reflexionsseite 42 des zweiten Prismas 34 angeordnet, dass ein Abstand zwischen der ersten Eintrittsseite 36 des ersten Prismas 32 und der Reflexionsseite 42 des zweiten Prismas 34 in der ersten Teilfläche 62 stets größer ist als ein entsprechender Abstand zwischen der ersten Eintrittsseite 36 und der Reflexionsseite 42 in der zweiten Teilfläche 64. Mit anderen Worten liegt also die zweite Teilfläche 64 der ersten Eintrittsseite 36 durchgängig näher als die erste Teilfläche 62.

Es ist ferner insbesondere vorgesehen, dass sich die erste und die zweite Teilfläche 62, 64, anders als in Fig. 4 dargestellt, überlappen. In einem solchen Ausführungsbeispiel ist ferner beispielsweise vorgesehen, dass aus einem Objektraum 11 einfallende erste Lichtstrahlen in der ersten Teilfläche 62 der Reflexionsfläche 42 reflektiert und in den linken Linsensystemkanal 48L eingekoppelt werden. Entsprechend werden aus dem Objektraum 11 einfallende zweite Lichtstrahlen in der zweiten Teilfläche 64 der Reflexionsfläche 42 reflektiert und in den rechten Linsensystemkanal 48R eingekoppelt.

Bei einem Verfahren zum Herstellen eines optischen Systems 20 eines Stereo-Videoendoskops 2 mit fester seitlicher Blickrichtung, welches eine seitwärtsblickende distale optische Baugruppe 24 und eine proximale optische Baugruppe 26 umfasst, werden das erste und das zweite Prisma 32, 34 der Umlenkprismengruppe 30 der distalen optischen Baugruppe 24 so ausgewählt oder angeordnet, dass die erste Eintrittsseite 36 des ersten Prismas 32 und die Reflexionsseite 42 des zweiten Prismas 34 einen Winkel $\alpha$ einschließen, der größer als der Totalreflexionswinkel $\theta_C$ des zweiten Prismas 34 ist.

Diese Auswahl und Anordnung umfasst nicht nur die geometrische Ausgestaltung des ersten und zweiten Prismas 32, 34 und deren Anordnung im optischen System 20, sondern auch die Auswahl der zur Herstellung dieser Prismen 32, 34 verwendeten Materialien bzw. Gläser, welche den jeweiligen Brechungsindex der Prismen 32, 34 bestimmen.

Bei einem Verfahren zum Reparieren eines Stereo-Videoendoskops 2 mit seitlicher Blickrichtung wird ähnlich verfahren. Es wird beispielsweise die Umlenkprismengruppe 30 eines herkömmlichen optischen Systems 20 durch eine Umlenkprismengruppe 30 ersetzt, welche die oben genannten Anforderungen erfüllt. Ebenso ist es möglich, die vollständige distale optische Baugruppe 24 oder gar das optische System 20 komplett auszutauschen.

Im Rahmen der Erfindung sind Merkmale, die mit "insbesondere" oder "vorzugsweise" gekennzeichnet sind, als fakultative Merkmale zu verstehen.

Bezugszeichenliste

| | |
|---|---|
| 2 | Stereo-Videoendoskop |
| 4 | Handgriff |
| 6 | Endoskopschaft |
| 8 | distale Spitze |
| 10 | Eintrittsfenster |
| 11 | Objektraum |
| 12 | distaler Abschnitt |
| 14 | Drehrad |
| 20 | optisches System |
| 22 | optische Achse |
| 24 | distale optische Baugruppe |
| 26 | proximale optische Baugruppe |
| 28 | Eintrittslinse |
| 30 | Umlenkprismengruppe |
| 32 | erstes Prisma |
| 34 | zweites Prisma |
| 36 | erste Eintrittsseite |
| 38 | erste Austrittsseite |
| 40 | zweite Eintrittsseite |
| 42 | Reflexionsseite |
| 44 | zweite Austrittsseite |
| 46 | Austrittslinse |
| 48L | linker Linsensystemkanal |
| 48R | rechter Linsensystemkanal |
| 50L | linke Linsengruppe |
| 50R | rechte Linsengruppe |
| 52L | linker Bildsensor |
| 52R | rechter Bildsensor |
| 54L | Sichtfeld des linken Kanals |
| 54R | Sichtfeld des rechten Kanals |
| 56 | Lichtstrahl |
| 58 | B-Down-Bereich |
| 60L | linke Blende |
| 60R | rechte Blende |
| 62 | erste Teilfläche |
| 64 | zweite Teilfläche |

| | |
|---|---|
| L | Längserstreckungsrichtung |
| $\alpha$ | Winkel |
| $\theta_C$ | Totalreflexionswinkel |

**Patentansprüche**

1. Optisches System (20) eines Stereo-Videoendoskops (2) mit fester seitlicher Blickrichtung, umfassend eine seitwärts blickende distale optische Baugruppe (24) und eine proximale optische Baugruppe (26), wobei die proximale optische Baugruppe (26) einen linken Linsensystemkanal (48L) und einen rechten Linsensystemkanal (48R) umfasst, die gleichartig aufgebaut sind, und wobei die distale optische Baugruppe dazu eingerichtet ist, aus einem Objektraum (11) einfallendes Licht in den linken Linsensystemkanal (48L) und in den rechten Linsensystemkanal (48R) der proximalen optischen Baugruppe (26) einzukoppeln, und wobei die distale optische Baugruppe (24) in einer Lichteinfallsrichtung aufeinanderfolgend eine Eintrittslinse (28), eine Umlenkprismengruppe (30) und eine Austrittslinse (46) umfasst, wobei die Umlenkprismengruppe (30) in Lichteinfallsrichtung aufeinanderfolgend ein erstes Prisma (32) und ein zweites Prisma (34) umfasst, wobei das erste Prisma (32) eine erste Eintrittsseite (36) und eine gegenüber dieser geneigte erste Austrittsseite (38) umfasst, und wobei das zweite Prisma (34) eine zweite Eintrittsseite (40), eine Reflexionsseite (42) und eine zweite Austrittsseite (44) umfasst, wobei die erste Eintrittsseite (36) und die Reflexionsseite (42) einen Winkel $(\alpha)$ einschließen, der größer als ein Totalreflexionswinkel $(\theta_C)$ des zweiten Prismas (34) ist, wobei eine erste Teilfläche (62) der Reflexionsseite (42) des zweiten Prismas (34) mit einer reflektierenden Schicht versehen ist und eine zweite Teilfläche (64) der Reflexionsseite (42) wahlweise unbeschichtet ist oder mit einer Antireflexbeschichtung versehen ist, wobei sich die erste und die zweite Teilfläche (62, 64) zur Gesamtfläche der Reflexionsseite (42) ergänzen, **dadurch gekennzeichnet, dass** ein Abstand zwischen der ersten Eintrittsseite (36) und der Reflexionsseite (42) in der ersten Teilfläche (62) stets größer ist als ein Abstand zwischen der ersten Eintrittsseite (36) und der Reflexionsseite (42) in der zweiten Teilfläche (64).

2. Optisches System (20) nach Anspruch 1, **dadurch gekennzeichnet, dass** das optische System (20) derart eingerichtet ist, dass aus einem Blickfeld (54L, 54R) des optischen Systems (20) einfallende Lichtstrahlen unter einem Winkel auf die Reflexionsseite (42) des zweiten Prismas (34) treffen, der größer als der Totalreflexionswinkel ist.

3. Optisches System (20) nach Anspruch 2, **dadurch gekennzeichnet, dass** das optische System (20) zumindest eine Blende (60L, 60R) umfasst, die das Blickfeld (54L, 54R) des optischen Systems (20) begrenzt, wobei der Winkel $(\alpha)$ zwischen der ersten Eintrittsseite (36) und der Reflexionsseite (42) unter Berücksichtigung eines Materials des zweiten Prismas (34), welches einen Brechungsindex (u2) und somit einen Totalreflexionswinkel $(\theta_C)$ festlegt, und unter Berücksichtigung eines maximalen Blickwinkels, der durch das Blickfeld (54L, 54R) festgelegt ist, derart eingestellt ist, dass für alle aus dem Blickfeld 54L, 54R) einfallenden Lichtstrahlen an der Reflexionsseite (42) des zweiten Prismas (34) Totalreflexion stattfindet.

4. Optisches System (20) nach einem der Ansprüche 1 bis 3, **dadurch gekennzeichnet, dass** aus einem Objektraum (11) einfallende erste Lichtstrahlen in der ersten Teilfläche (62) der Reflexionsfläche (42) reflektiert und in den linken Linsensystemkanal (48L) eingekoppelt werden und aus dem Objektraum (11) einfallende zweite Lichtstrahlen in der zweiten Teilfläche (64) der Reflexionsfläche (42) reflektiert und in den rechten Linsensystemkanal (48R) eingekoppelt werden.

5. Stereo-Videoendoskop (2) mit fester seitlicher Blickrichtung, **gekennzeichnet durch** ein optisches System (20) nach einem der Ansprüche 1 bis 4.

6. Verfahren zum Herstellen eines optischen Systems (20) eines Stereo-Videoendoskops (2) mit fester seitlicher Blickrichtung, wobei das Stereo-Videoendoskop (2) eine seitwärts blickende distale optische Baugruppe (24) und eine proximale optische Baugruppe (26) umfasst, und wobei die proximale optische Baugruppe (26) einen linken Linsensystemkanal (48L) und einen rechten Linsensystemkanal (48R) umfasst, die gleichartig aufgebaut sind, und wobei die distale optische Baugruppe (24) dazu eingerichtet ist, aus einem Objektraum (11) einfallendes Licht in den linken Linsensystemkanal (48L) und in den rechten Linsensystemkanal (48R) der proximalen optischen Baugruppe (26) einzukoppeln, und wobei die distale optische Baugruppe (24) in einer Lichteinfallsrichtung aufeinanderfolgend eine Eintrittslinse (28), eine Umlenkprismengruppe (30) und eine Austrittslinse (46) umfasst, wobei die Umlenkprismengruppe (30) in Lichteinfallsrichtung aufeinanderfolgend ein erstes Prisma (32) und ein zweites Prisma (34) umfasst, wobei das erste Prisma (32) eine erste Eintrittsseite (36) und eine gegenüber dieser geneigte erste Austrittsseite (38) umfasst, und wobei das zweite Prisma (34) eine zweite Eintrittsseite (40), eine Reflexionsseite (42) und eine zweite Austrittsseite (44) umfasst, wobei das erste und das zweite Prisma (32, 34) so ausgewählt oder angeordnet werden, dass die erste Eintrittsseite (36) und die Reflexionsseite (42) einen Winkel $(\alpha)$ einschließen, der größer als ein Totalreflexionswinkel $(\theta_C)$ des zweiten Prismas (34) ist, wobei eine erste Teilfläche (62) der Reflexionsseite (42) des zweiten Prismas (34) mit einer reflektierenden Schicht versehen wird und eine zweite Teilfläche (64) der Reflexionsseite (42) wahlweise unbeschichtet belassen wird oder mit einer Antireflexbeschichtung versehen wird, wobei sich die erste und die zweite Teilfläche (62, 64) zur Gesamtfläche der Reflexionsseite (42) ergänzen, **dadurch gekennzeichnet, dass** ein Abstand zwischen der ersten Eintrittsseite (36) und der Reflexionsseite (42) in der ersten Teilfläche (62) stets größer ist als ein Abstand zwischen der ersten Eintrittsseite (36) und der Reflexionsseite (42) in der zweiten Teilfläche (64).

7. Verfahren nach Anspruch 6, **dadurch gekenn-**

zeichnet, dass das optische System (20) mit zumindest einer Blende (60L, 60R) versehen wird, die das Blickfeld (54L, 54R) des optischen Systems (20) begrenzt, wobei der Winkel ($\alpha$) zwischen der ersten Eintrittsseite (36) und der Reflexionsseite (42) unter Berücksichtigung eines Materials des zweiten Prismas (34), welches einen Brechungsindex (u2) und somit einen Totalreflexionswinkel ($\theta_C$) festlegt, und unter Berücksichtigung eines maximalen Blickwinkels, der durch das Blickfeld (54L, 54R) festgelegt ist, derart eingestellt wird, dass für alle aus dem Blickfeld (54L, 54R) einfallenden Lichtstrahlen an der Reflexionsseite (42) des zweiten Prismas (34) Totalreflexion stattfindet.

8. Verfahren nach Anspruch 7, **dadurch gekennzeichnet, dass** die Reflexionsseite (42) des zweiten Prismas (34) wahlweise unbeschichtet belassen wird oder mit einer Antireflexbeschichtung versehen wird.

9. Verfahren zum Reparieren eines Stereo-Videoendoskops (2) mit seitlicher Blickrichtung, wobei ein optisches System (20) des Stereo-Videoendoskops (2) eine seitwärts blickende distale optische Baugruppe (24) und eine proximale optische Baugruppe (26) umfasst, und wobei die proximale optische Baugruppe (26) einen linken Linsensystemkanal (48L) und einen rechten Linsensystemkanal (48R) umfasst, die gleichartig aufgebaut sind, und wobei die distale optische Baugruppe (24) dazu eingerichtet ist, aus einem Objektraum (11) einfallendes Licht in den linken Linsensystemkanal (48L) und in den rechten Linsensystemkanal (48R) der proximalen optischen Baugruppe (26) einzukoppeln, und wobei die distale optische Baugruppe (24) in einer Lichteinfallsrichtung aufeinanderfolgend eine Eintrittslinse (28), eine Umlenkprismengruppe (30) und eine Austrittslinse (46) umfasst, wobei die Umlenkprismengruppe (30) in Lichteinfallsrichtung aufeinanderfolgend ein erstes Prisma (32) und ein zweites Prisma (34) umfasst, wobei das erste Prisma (32) eine erste Eintrittsseite (36) und eine gegenüber dieser geneigte erste Austrittsseite (38) umfasst, und wobei das zweite Prisma (34) eine zweite Eintrittsseite (40), eine Reflexionsseite (42) und eine zweite Austrittsseite (44) umfasst, wobei die Umlenkprismengruppe (30) ausgetauscht und durch eine neue Umlenkprismengruppe (30) ersetzt wird, bei der das erste und das zweite Prisma (32, 34) so ausgewählt oder angeordnet werden, dass die erste Eintrittsseite (36) und die Reflexionsseite (42) einen Winkel ($\alpha$) einschließen, der größer als ein Totalreflexionswinkel ($\theta_C$) des zweiten Prismas (34) ist, wobei eine erste Teilfläche (62) der Reflexionsseite (42) des zweiten Prismas (34) mit einer reflektierenden Schicht versehen wird und eine zweite Teilfläche (64) der Reflexionsseite (42) wahlweise unbeschichtet belassen wird oder mit einer Antireflexbeschichtung versehen wird, wobei sich die erste und die zweite Teilfläche (62, 64) zur Gesamtfläche der Reflexionsseite (42) ergänzen, **dadurch gekennzeichnet, dass** ein Abstand zwischen der ersten Eintrittsseite (36) und der Reflexionsseite (42) in der ersten Teilfläche (62) stets größer ist als ein Abstand zwischen der ersten Eintrittsseite (36) und der Reflexionsseite (42) in der zweiten Teilfläche (64).

## Claims

1. An optical system (20) of a stereo video endoscope (2) with a fixed lateral viewing direction, comprising a laterally-viewing distal optical assembly (24) and a proximal optical assembly (26), wherein the proximal optical assembly (26) comprises a left lens system channel (48L) and a right lens system channel (48R) that are designed similarly, and wherein the distal optical assembly is configured to couple incident light from an object space (11) into the left lens system channel (48L) and into the right lens system channel (48R) of the proximal optical assembly (26), and wherein the distal optical assembly (24) sequentially comprises an entrance lens (28), a deflection prism group (30) and an exit lens (46) in a direction of incident light, wherein the deflection prism group (30) sequentially comprises a first prism (32) and a second prism (34) in the direction of incident light, wherein the first prism (32) comprises a first entrance side (36) and a first exit side (38) at an angle relative thereto, and wherein the second prism (34) comprises a second entrance side (40), a reflection side (42) and a second exit side (44), wherein the first entrance side (36) and the reflection side (42) enclose an angle ($\alpha$) that is greater than a total reflection angle ($\theta_C$) of the second prism (34), wherein a first partial surface (62) of the reflection side (42) of the second prism (34) is provided with a reflective coating, and a second partial surface (64) of the reflection side (42) is optionally uncoated or is provided with an anti-reflection coating, wherein the first and the second partial surfaces (62, 64) supplement each other to form the entire surface of the reflection side (42), **characterized in that** a distance between the first entrance side (36) and the reflection side (42) in the first partial surface (62) is always larger than the distance between the first entrance side (36) and the reflection side (42) in the second partial surface (64).

2. The optical system (20) according to claim 1, **characterized in that** the optical system (20) is configured such that incident light beams from a field of view (54L, 54R) of the optical system (20) contact the reflection side (42) of the second prism (34) at an angle that is greater than the total reflection angle.

3. The optical system (20) according to claim 2, **characterized in that** the optical system (20) comprises at least one aperture (60L, 60R) that borders the field of view (54L, 54R) of the optical system (20), wherein the angle ($\alpha$) between the first entrance side (36) and the reflection side (42) is adjusted taking into account a material of the second prism (34) that establishes a refraction index (u2) and hence a total reflection angle ($\theta_C$), and taking into account a maximum viewing angle that is established by the field of view (54L, 54R) such that total reflection of all incident light beams from the field of view ( 54L, 54R) occurs at the reflection side (42) of the second prism (34).

4. The optical system (20) according to one of claims 1 to 3, **characterized in that** incident first light beams from the object space (11) are reflected at the first partial surface (62) of the reflective surface (42) and are coupled into the left lens system channel (48L), and incident second light beams from the object space (11) are reflected at the second partial surface (64) of the reflective surface (42) and coupled into the right lens system channel (48R).

5. A stereo video endoscope (2) with a fixed lateral viewing direction, **characterized by** an optical system (20) according to one of claims 1 to 4.

6. A method for manufacturing of an optical system (20) of a stereo video endoscope (2) with a fixed lateral viewing direction, wherein the stereo video endoscope (2) comprises a laterally-viewing distal optical assembly (24) and a proximal optical assembly (26), and wherein the proximal optical assembly (26) comprises a left lens system channel (48L) and a right lens system channel (48R) that are designed similarly, and wherein the distal optical assembly (24) is configured to couple incident light from an object space (11) into the left lens system channel (48L) and into the right lens system channel (48R) of the proximal optical assembly (26), and wherein the distal optical assembly (24) sequentially comprises an entrance lens (28), a deflection prism group (30) and an exit lens (46) in a direction of incident light, wherein the deflection prism group (30) sequentially comprises a first prism (32) and a second prism (34) in the direction of incident light, wherein the first prism (32) comprises a first entrance side (36) and a first exit side (38) at an angle relative thereto, and wherein the second prism (34) comprises a second entrance side (40), a reflection side (42) and a second exit side (44), wherein the first and the second prism (32, 34) are selected or arranged such that the first entrance side (36) and the reflection side (42) enclose an angle ($\alpha$) that is greater than a total reflection angle ($\theta_C$) of the second prism (34), wherein a first partial surface (62) of the reflection side (42) of the second prism (34) is provided with a reflective coating, and a second partial surface (64) of the reflection side (42) is optionally left uncoated or is provided with an anti-reflection coating, wherein the first and the second partial surfaces (62, 64) supplement each other to form the entire surface of the reflection side (42), **characterized in that** a distance between the first entrance side (36) and the reflection side (42) in the first partial surface (62) is always larger than the distance between the first entrance side (36) and the reflection side (42) in the second partial surface (64).

7. The method according to claim 6, **characterized in that** the optical system (20) is provided with at least one aperture (60L, 60R) that borders the field of view (54L, 54R) of the optical system (20), wherein the angle ($\alpha$) between the first entrance side (36) and the reflection side (42) is adjusted taking into account a material of the second prism (34) that establishes a refraction index (u2) and hence a total reflection angle ($\theta_C$), and taking into account a maximum viewing angle that is set by the field of view (54L, 54R) such that total reflection of all incident light beams from the field of view ( 54L, 54R) occurs at the reflection side (42) of the second prism (34).

8. The method according to claim 7, **characterized in that** the reflection side (42) of the second prism (34) is optionally left uncoated, or is provided with an anti-reflection coating.

9. A method for repairing of a stereo video endoscope (2) with a lateral viewing direction, wherein an optical system (20) of the stereo video endoscope (2) comprises a laterally-viewing distal optical assembly (24) and a proximal optical assembly (26), and wherein the proximal optical assembly (26) comprises a left lens system channel (48L) and a right lens system channel (48R) that are designed similarly, and wherein the distal optical assembly (24) is configured to couple incident light from an object space (11) into the left lens system channel (48L) and into the right lens system channel (48R) of the proximal optical assembly (26), and wherein the distal optical assembly (24) sequentially comprises an entrance lens (28), a deflection prism group (30) and an exit lens (46) in a direction of incident light, wherein the deflection prism group (30) sequentially comprises a first prism (32) and a second prism (34) in the direction of incident light, wherein the first prism (32) comprises a first entrance side (36) and a first exit side (38) at an angle relative thereto, and wherein the second prism (34) comprises a second entrance side (40), a reflection side (42) and a second exit side (44), wherein the deflection prism group (30) is exchanged and replaced by a new deflection prism group (30), wherein the first and the second prism (32, 34) are selected or arranged such that the first entrance side (36) and the reflection side (42) enclose an angle ($\alpha$) that is greater than a total reflection angle ($\theta_C$) of the second prism (34), wherein a first partial surface (62) of the reflection side (42) of the second prism (34) is provided with a reflective coating, and a second partial surface (64) of the reflection side (42) is optionally left uncoated or is provided with an anti-reflection coating, wherein the first and the second partial surfaces (62, 64) supplement each other to form the entire surface of the reflection side (42), **characterized in that** a distance between the first entrance side (36) and the reflection side (42) in the first partial surface (62) is always larger than the distance between the first entrance side (36) and the reflection side (42) in the second partial surface (64).

## Revendications

1. Système optique (20) d'un vidéo-endoscope stéréo (2) à direction de vision latérale fixe, comprenant un ensemble optique distal (24) à vision orientée latéralement et un ensemble optique proximal (26), l'ensemble optique proximal (26) comprenant un canal gauche (48L) de système de lentilles et un canal droit (48R) de système de lentilles qui sont de conception identique, et l'ensemble optique distal étant conçu de façon à relier la lumière incidente provenant d'un espace objet (11) dans le canal gauche (48L) de système de lentilles et dans le canal droit (48R) de système de lentilles de l'ensemble optique proximal (26), et l'ensemble optique distal (24) comprenant, successivement dans une direction d'incidence de la lumière, une lentille d'entrée (28), un groupe de prismes de déviation (30) et une lentille de sortie (46), le groupe de prismes de déviation (30) comprenant, successivement dans la direction d'incidence de la lumière, un premier prisme (32) et un deuxième prisme (34), le premier prisme (32) comprenant une première face d'entrée (36) et une première face de sortie (38) inclinée par rapport à celle-ci, et le deuxième prisme (34) comprenant une deuxième face d'entrée (40), une face de réflexion (42) et une deuxième face de sortie (44), la première face d'entrée (36) et la face de réflexion (42) formant un angle ($\alpha$) supérieur à l'angle de réflexion totale ($\theta c$) du deuxième prisme (34), une première surface partielle (62) de la face de réflexion (42) du deuxième prisme (34) étant pourvue d'une couche réfléchissante et une deuxième surface partielle (64) de la face de réflexion (42) étant au choix laissée non revêtue ou étant pourvue d'un revêtement antireflet, les première et deuxième surfaces partielles (62, 64) s'ajoutant à la surface totale de la face de réflexion (42), **caractérisé en ce qu'**une distance entre la première face d'entrée (36) et la face de réflexion (42) dans la première surface partielle (62) est toujours supérieure à une distance entre la première face d'entrée (36) et la face de réflexion (42) dans la deuxième surface partielle (64).

2. Système optique (20) selon la revendication 1, **caractérisé en ce que** le système optique (20) est conçu de telle sorte que les rayons lumineux incidents provenant d'un champ de vision (54L, 54R) du système optique (20) frappent la face de réflexion (42) du deuxième prisme (34) sous un angle qui est supérieur à l'angle de réflexion totale.

3. Système optique (20) selon la revendication 2, **caractérisé en ce que** le système optique (20) comprend au moins un diaphragme (60L, 60R) qui limite le champ de vision (54L, 54R) du système optique (20), l'angle ($\alpha$) entre la première face d'entrée (36) et la face de réflexion (42) étant réglé en tenant compte d'un matériau du deuxième prisme (34) qui détermine un indice de réfraction (u2), et donc un angle de réflexion totale ($\theta c$), et en tenant compte d'un angle de vision maximal qui est déterminé par le champ de vision (54L, 54R) de telle sorte qu'une réflexion totale se produise sur la face de réflexion (42) du deuxième prisme (34) pour tous les rayons lumineux incidents provenant du champ de vision (54L, 54R).

4. Système optique (20) selon l'une des revendications 1 à 3, **caractérisé en ce que** des premiers rayons lumineux provenant d'un espace objet (11) sont réfléchis dans la première surface partielle (62) de la face de réflexion (42) et sont reliés dans le canal gauche (48L) de système de lentilles, et **en ce que** des deuxièmes rayons lumineux provenant de l'espace objet (11) sont réfléchis dans la deuxième surface partielle (64) de la face de réflexion (42) et sont reliés dans le canal droit (48R) de système de lentilles.

5. Vidéo-endoscope stéréo (2) à direction de vision latérale fixe, **caractérisé par** un système optique (20) selon l'une des revendications 1 à 4.

6. Procédé de fabrication d'un système optique (20) d'un vidéo-endoscope stéréo (2) à direction de vision latérale fixe, le vidéo-endoscope stéréo (2) comprenant un ensemble optique distal (24) à vision orientée latéralement et un ensemble optique proximal (26), l'ensemble optique proximal (26) comprenant un canal gauche (48L) de système de lentilles et un canal droit (48R) de système de lentilles de conception identique, et dans lequel l'ensemble optique distal (24) est conçu pour relier la lumière incidente provenant d'un espace objet (11) dans le canal gauche (48L) de système de lentilles et dans le canal droit (48R) de système de lentilles de l'ensemble optique proximal (26), et l'ensemble optique distal (24) comprenant successivement, dans le sens d'incidence de la lumière, une lentille d'entrée (28), un groupe de prismes de déviation (30) et une lentille de sortie (46), le groupe de prismes de déviation (30) comprenant, successivement dans la direction d'incidence de la lumière, un premier prisme (32) et un deuxième prisme (34), le premier prisme (32) comprenant un première face d'entrée (36) et une première face de sortie (38) inclinée par rapport à celle-ci, et le deuxième prisme (34) comprenant une deuxième face d'entrée (40), une face de réflexion (42) et une deuxième face de sortie (44), le premier et le deuxième prismes (32, 34) étant choisis ou agencés de telle sorte que la première face d'entrée (36) et la face de réflexion (42) forment un angle ($\alpha$) supérieur à l'angle de réflexion totale ($\theta c$) du deuxième prisme (34), une première surface partielle (62) de la face de réflexion (42) du deuxième prisme (34) étant pourvue d'une couche réfléchissante et une deuxième surface partielle (64) de la face de réflexion (42) étant au choix laissée non revêtue ou étant pourvue d'un revêtement antireflet, les première et deuxième surfaces partielles (62, 64) se complétant pour former la surface totale de la face de réflexion (42), **caractérisé en ce que** la distance entre la première face d'entrée (36) et la face de réflexion (42) dans la première surface partielle (62) est toujours supérieure à la distance entre la première face d'entrée (36) et la face de réflexion (42) dans la deuxième surface partielle (64).

7. Procédé selon la revendication 6, **caractérisé en ce que** le système optique (20) est pourvu d'au moins un diaphragme (60L, 60R) qui limite le champ de vision (54L, 54R) du système optique (20), l'angle ($\alpha$) entre la première face d'entrée (36) et la face de réflexion (42) étant ajusté en tenant compte d'un matériau du deuxième prisme (34) qui détermine un indice de réfraction (u2) et donc un angle de réflexion totale ($\theta c$), et en tenant compte d'un angle de vision maximal qui est déterminé par le champ de vision (54L, 54R) de telle sorte qu'une réflexion totale se produise sur la face de réflexion (42) du deuxième prisme (34) pour tous les rayons lumineux incidents provenant du champ de vision (54L, 54R).

8. Procédé selon la revendication 7, **caractérisé en ce que** la face de réflexion (42) du deuxième prisme (34) est au choix laissée non revêtue ou est pourvue d'un revêtement antireflet.

9. Procédé de réparation d'un vidéo-endoscope stéréo (2) à direction de vision latérale, dans lequel un système optique (20) du vidéo-endoscope stéréo (2) comprend un ensemble optique distal (24) à vision orientée latéralement et un ensemble optique proximal (26), et l'ensemble optique proximal (26) comprend un canal gauche (48L) de système de lentilles et un canal droit (48R) de système de lentilles qui sont de construction identique, l'ensemble optique distal (24) étant conçu de façon à relier la lumière incidente provenant d'un espace objet (11) dans le canal gauche (48L) de système de lentilles et dans le canal droit (48R) de système de lentilles de l'ensemble optique proximal (26), et l'ensemble optique distal (24) comprenant, successivement dans une direction d'incidence de la lumière, une lentille d'entrée (28), un groupe de prismes de déviation (30) et une lentille de sortie (46), le groupe de prismes de déviation (30) comprenant, successivement dans la direction d'incidence de la lumière, un premier prisme (32) et un deuxième prisme (34), le premier prisme (32) comprenant un première face d'entrée (36) et une première face de sortie (38) inclinée par rapport à celle-ci, et le deuxième prisme (34) comprenant une deuxième face d'entrée (40), une face de réflexion (42) et une deuxième face de sortie (44), le groupe de prismes de déviation (30) étant remplacé par un nouveau groupe de prismes de déviation (30), le premier et le deuxième prismes (32, 34) étant choisis ou agencés de telle sorte que la première face d'entrée (36) et la face de réflexion (42) forment un angle ($\alpha$) supérieur à l'angle de réflexion totale ($\theta c$) du deuxième prisme (34), une première surface partielle (62) de la face de réflexion (42) du deuxième prisme (34) étant pourvue d'une couche réfléchissante et une deuxième surface partielle (64) de la face de réflexion (42) étant au choix laissée non revêtue ou étant pourvue d'un revêtement antireflet, les première et deuxième surfaces partielles (62, 64) se complétant pour former la surface totale de la face de réflexion (42), **caractérisé en ce qu'**une distance entre la première face d'entrée (36) et la face de réflexion (42) dans la première surface partielle (62) est toujours supérieure à une distance entre la première face d'entrée (36) et la face de réflexion (42) dans la deuxième surface partielle (64).

Fig. 1

Fig. 2

## Fig. 3

## Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014206513 A1
- DE 102013215422 A1
- EP 2730210 A1
- US 5861987 A
- JP H09288240 A